# EUROPEAN PATENT APPLICATION

(11) **EP 1 913 884 A1**
(43) Date of publication of application: **23.04.2008**
(21) Application number: 05777237.8
(22) Date of filing: 04.08.2005
(51) Int. Cl.: A61B 17/70

(54) **DIGITISED SPINAL SYSTEM**

(71) Applicant: Arvizo Arvizo, Aldo, C.P. 31200 Chihuahua, Chihuahua (MX)
(72) Inventor: Arvizo Arvizo, Aldo, C.P. 31200 Chihuahua, Chihuahua (MX)
(74) Representative: Lorente Berges, Ana
(86) International application number: PCT/MX2005/000065
(87) International publication number: WO 2007/015634

(57) **Abstract**

This invention referred to herein, is a biocompatible metal piece intended for stabilizing vertebral bodies in cervical, thoracic and lumbar regions. This piece remarkably diminishes the profile, weigh and volume of an implant; the compatibility of the curving digitalized surfaces allow to immobilize vertebral bodies without internal efforts, achieving an important diminishing of time during the implant installation.

## Description

### OBJECTIVE

The invention referred to herein is whole new piece made out of metal biocompatible with curving digitalized surfaces which basically allows vertebral bodies to maintain themselves stable in cervical, thoracic and lumbar regions.

### BACKGROUNG OF THE INVENTION

Nowadays, several methods to stabilize vertebral pieces are utilized. Some of these include a number of cylindrical universal bars joined by a variety of fastening mechanic systems which hold vertebral bodies still; however, these systems have the following disadvantages:

Big implants include a great number of pieces assembled with complicated manual tools which produces internal efforts to pre-form the bars at room temperature, a task that increases the weight and also the time needed to perform the assembly on several components during surgical operations.

With the end of avoiding such inconveniences, these pieces have been developed with a digitalized biocompatible metal, an invention that is intended to be protected by means of this written requirement. Since such pieces made out of digitalized biocompatible metal with curving surfaces remarkably diminish the profile, weight and volume of the implant and because the compatibility of curving digitalized surfaces do not demand the use of special tools whatsoever, this invention offers an advantage that allows alignments without internal efforts achieving an important diminishing of time during surgical operations.

### BRIEF DESCRIPTION OF THE DRAWINGS.

The details that depict these new elements are shown in the following description, along with the drawings attached herein with the illustrations. For reference purposes, the points shown in each detail are also shown in the parts.
In figure 1, a perspective top view of the conventional digitalized blocompalible metal part is shown. Three circular orifices can be seen from there (1), such orifices are intended as the receptors for fixing oriented screws. A bar made out of biocompatible metal with curving surfaces is also shown (2).
In figure 2, a biocompatible metal bar is shown (2), with perforations for its assembly (1); the digitalized curves are similar to those of the transverse apophysis on the fixing surface (3).
Figure 3 shows a longitudinal view. This illustration shows the orifices made for its assembly (1) and the digitalized biocompatible metal piece (2), and the view of the digitalized curves (3).
Figure 4 shows a conventional perspective of the biocompatible metal bar. This view also shows the fixing oriented orifices (1), the body of the biocompatible metal bar (2) and the digitalized surfaces (3).

### DETAILED DESCRIPTION OF THE INVENTION.

To complement the description of this invention and for a better understanding of its characteristics, some drawings are included herein with unlimited and illustrative purposes, showing the following:

### ATTAINMENT OF THE INVENTION.

These figures illustrate important areas of the digitalized biocompatible metal pieces.

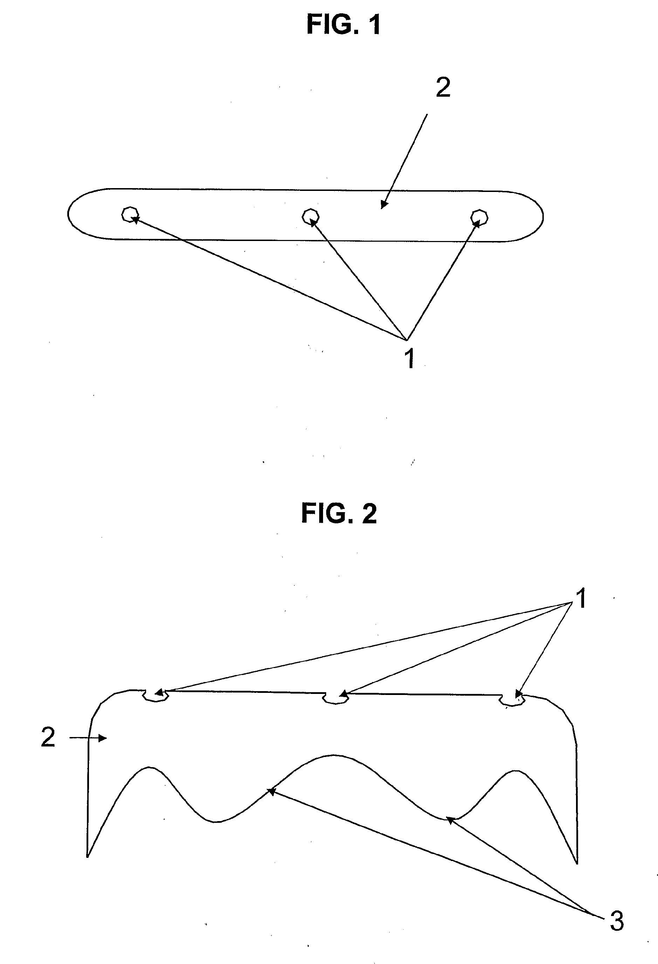

Figure 1 shows a bar (2), which consists of a solid piece made out of biocompatible metal, the bar has a rectangular shape with 4 rounded corners and flat on each side. It also shows three orifices designed as oriented fixing screw receptors (1), which pass through this biocompatible solid metal bar (2).

Figure 2 shows the body of a biocompatible metal bar (2), limited on its parallel edges, it has a rectangular shape, its edges are rounded at the top and the surface at the bottom has digitalized curves (3), such curves have the same shape as the transverse apophysis of vertebral bodies.

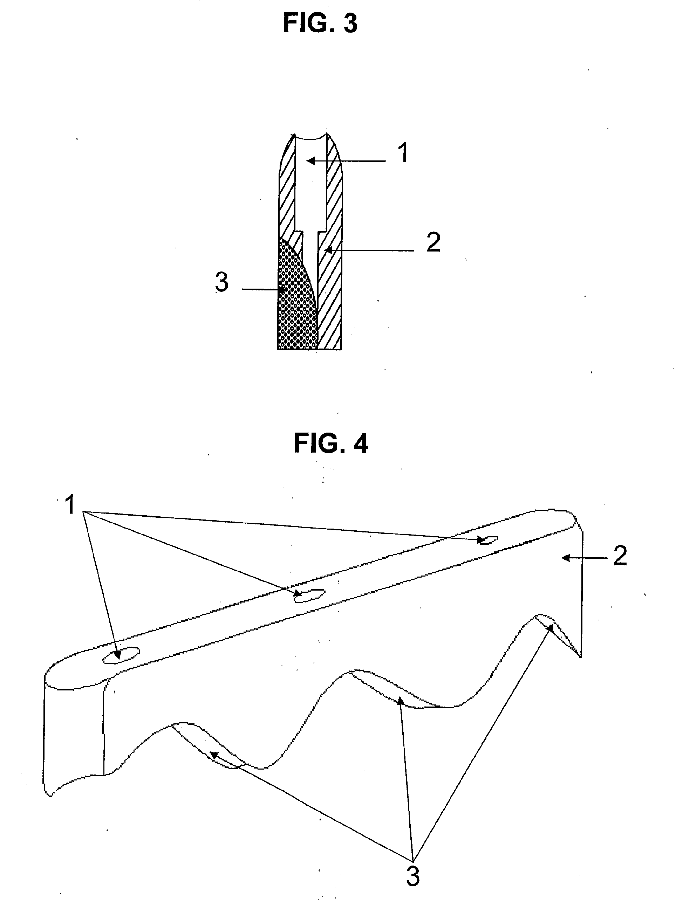

Figure 3 also shows a cross section of the solid digitalized metal bar, showing the perforations made for its assembly (1), which are cylindrical with two diameters that pass through the biocompatible metal bar (2) it also illustrates a cross section view of the curves on the transverse apophysis of the vertebras.

Figure 4 is a perspective conventional view of the digitalized bar that clearly shows the oriented fixing orifices (1), the body of the biocompatible metal bar (2) and the digitalized surfaces (3) which are in contact with the transverse apophysis of the vertebras. In virtue of the above, having depicted my invention in detail and comprehensibly, I consider it a novelty, therefore, I claim the exclusive property of rights on the same according to the following clauses:

## Claims

1. This piece with digitalized surfaces is featured by a sole rectangular solid metal bar, rounded by its edges on which surfaces of contact have curves that are generated according to the curves of the transverse apophysis on vertebral parts.

2. By producing identical cavities between the digitalized bars and transverse apophysis of the vertebral bodies, this piece makes possible that all the efforts may be distributed uniformly in a tridimensional manner, avoiding movements everywhere; the outcome is a perfectly fixed assembly between the parts.

3. The invention is also featured by perforations that should fix the digitalized pieces to the vertebras. Said perforations are located and oriented according to the real position of the vertebral pendiculous, which are fully observed in the tridimensional solid model.

4. This digitalized piece is **characterized by** transmitting efforts over all of the contact area in three dimensions.

5. The digitalized piece is also **characterized by** a remarkable diminishing of the profile, weigh and volume of an implant, which is custom designed for a particular patient.

6. The compatibility of the digitalized surfaces allows to perform a sole positioning over the vertebral bodies to be immobilized.

7. This piece is **characterized by** the generation of digitalized surfaces on biocompatible materials which specifically match the surfaces of the transverse apophysis; obtained on a functional position from a selected patient; the digitalized surfaces of the individual vertebral parts are generated on a solid bar made out of a biocompatible material in such a way that once when the parts are fixed, they become a sole unit.

## Amended claims

### Amended claims under Art. 19.1 PCT

In virtue of the above, having depicted my invention in detail and comprehensibly, I consider it a novelty, therefore, I claim the exclusive property of rights on the same according to the following clauses:
**1.** This piece with digitalized surfaces is featured by a sole rectangular solid metal bar, rounded by its edges on which surfaces of contact have curves that are generated according to the curves of the transverse apophysis on vertebral parts.
**2.** By producing Identical cavities between the digitalized bars and transverse apophysis of the vertebral bodies, this piece makes possible that all the efforts may be distributed uniformly in a tridimensional manner, avoiding movements everywhere; the outcome is a perfectly fixed assembly between the parts.
**3.** The invention is also featured by perforations that should fix the digitalized pieces to the vertebras. Said perforations are located and oriented according to the real position of the vertebral pendiculous, which are fully observed in the tridimensional solid model.
**4.** This digitalized piece is **characterized by** transmitting efforts over all of the contact area in three dimensions.
**5.** The digitalized piece is also **characterized by** a remarkable diminishing of the profile, weigh and volume of an implant, which is custom designed for a particular patient.
**6.** The compatibility of the digitalized surfaces allows to perform a sole positioning over the vertebral bodies to be immobilized.
**7.** This piece is **characterized by** the generation of digitalized surfaces on biocompatible materials which specifically match the surfaces of the transverse apophysis; obtained on a functional position from a selected patient; the digitalized surfaces of the individual vertebral parts are generated on a solid bar made out of a biocompatible material in such a way that once when the parts are fixed, they become a sole unit.
